# EUROPEAN PATENT SPECIFICATION

(11) **EP 3 456 385 B1**
(45) Date of publication and mention of the grant of the patent: **26.02.2020**
(21) Application number: 18194287.1
(22) Date of filing: 13.09.2018
(51) Int. Cl.: A61N 5/10, G21F 1/08, A61C 19/00

(54) **DEVICE FOR PROTECTING DENTAL ARCHES**
VORRICHTUNG ZUM SCHUTZ VON ZAHNBÖGEN
APAREIL DE PROTECTION DES ARCADES DENTAIRES

(30) Priority: 19.09.2017 IT 201700104641
(43) Date of publication of application: 20.03.2019
(73) Proprietor: Setti, Stefano, 41042 Spezzano di Fiorano (MO) (IT); Picenni, Elena, 24040 Verdellino (BG) (IT)
(72) Inventor: Setti, Stefano, 41042 Spezzano di Fiorano (MO) (IT); Picenni, Elena, 24040 Verdellino (BG) (IT)
(74) Representative: Ottazzo, Marco Francesco Agostino

(56) References cited:
- WO-A1-98/01865
- DE-U1- 8 416 884
- KR-A- 20170 094 849
- US-A1- 2012 012 120
- US-A1- 2013 131 427
- US-A1- 2015 082 614

## Description

The present invention refers to a device for protecting dental arches. More specifically, the present invention refers to a device for protecting dental arches during radiotherapy cycles.

More and more often disastrous consequences are experienced from radiotherapy in oral cavity and in throat, and specially on teeth. Many persons having undergone radiotherapy cycles subsequently experience severe problems in their teeth, because the ionizing radiations used destroy the dental tissues.

At present, there are no technical solutions that are suitable for protecting dental arches and paradental tissues from ionizing radiations. As a matter of fact, there are technical solutions that are suitable for protecting dental arches and paradental tissues from ionizing radiations only partially, for instance those disclosed in patent documents US 2012/012120 A1, US 2015/082614 A1, DE 8416884 U1, KR 2017-0094849 A, WO 98/01865 A1, US 2013/131427 A1, and US 2015/041685 A1.

An object of the present invention is thus to provide a device for protecting dental arches that provides an effective and total protection from ionizing radiations.

Another object of the present invention is to provide a device for protecting dental arches that can be manufactured according to a process that is standardized and customizable for any patients.

A further object of the present invention is to provide a device for protecting dental arches that, when used by a patient, bothers him/her as little as possible.

These objects and others, which will be apparent to those skilled in the art, are achieved by a device for protecting dental arches implemented according to the technical teachings of the attached claims.

This device for protecting dental arches comprises at least one arc-shaped body which covers a respective dental arch. Each arc-shaped body comprises in turn an arc-shaped flat wall, an outer wall, which transversally extends from an outer edge of said flat wall, and an inner wall, which transversally projects from an inner edge of said flat wall. The inner wall of a respective arc-shaped body is placed in front of the outer wall so as to form a tooth receiving groove. The inner wall of a respective arc-shaped body has a length that is equal to, or slightly greater than, the length of the teeth, so as to cover and protect their whole inner surface, as well as part of the gum immediately adjacent to the base of the teeth, to also protect the roots of the teeth.

Two arc-shaped bodies are advantageously provided, connected to each other at the ends of their respective flat walls, so as to allow for the two arc-shaped bodies to move one with respect to the other. The device in manufactured with a material comprising an ionizing radiation absorbent material. Advantageously the material used to manufacture the body of the device is fully made of an ionizing radiation absorbent material. Preferably, such material is lead.

Further characteristics and advantages of the device for protecting dental arches according to the present invention will be apparent from a description of a preferred but non-exclusive embodiment of such device for protecting dental arches, which is shown, for explanatory hence non-limitative purposes, in the attached drawing, wherein:

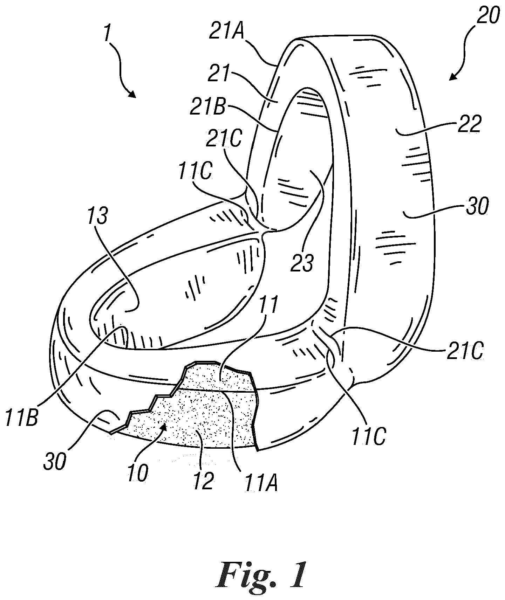

figure 1 is a perspective view of the device for protecting dental arches.

With reference to figure 1, a device 1 for protecting dental arches is shown. Such device 1 comprises at least one arc-shaped body 10, 20 configured for at least partially covering a respective dental arch.

For this purpose, each arc-shaped body 10, 20 comprises a respective arc-shaped flat wall 11, 21, configured for covering the ends of the teeth of a dental arch of a user of the device 1. The flat wall 11, 21, which is comprised in a plane and is substantially U-shaped, includes an outer edge 11A, 21A, which in use is oriented toward the outside of mouth, an inner edge 11B, 21B, which in use is oriented toward the inside of mouth, and two ends 11C, 21C, which in use are located at the ends of the dental arch (figure 1).

Each arc-shaped body 10, 20 also comprises an outer wall 12, 22 which transversally extends from the outer edge 11A, 21A of its respective flat wall 11, 21. This outer wall 12, 22 is configured for covering the outer surface of the teeth of a dental arch of a user of the device 1. In the context of the present invention, outer surface of the teeth means the surface of the teeth that is oriented toward the outside of mouth.

Preferably the outer wall 12, 22 extends in a direction substantially transversal to the plane of the flat wall 11, 21 and its length is such as to cover the complete outer surface of the teeth (from the upper end down to the base), and also preferably a portion of the paradental tissue immediately below the teeth themselves. This way, also the roots of the teeth are at least partially covered by the device 1, and consequently protected from ionizing radiations.

According to the present invention, each arc-shaped body 10, 20 is manufactured with a material that comprises an ionizing radiation absorbent material (indicated as a dotted area in figure 1). Making such device 1 from a material featuring ionizing radiation absorbing properties allows to obtain a device 1 that protects the teeth and paradental tissues from ionizing radiations. This protection is effective, because the device 1 features such a shape as to cover the end and the outer surface of all teeth of a patient. The device 1 is also easy to use, because its shape and dimensions are such as to make it suitable for being inserted inside a mouth. Also, such device 1 being made from this type of material allows to produce the device 1 according to a standard procedure, which is also well consolidated, and to have it manufactured with shapes and dimensions suitable for the patient who shall wear it.

It is preferred that each arc-shaped body 10, 20 be manufactured by using a material that is fully made from an ionizing radiation absorbent material. Even more preferably, such ionizing radiation absorbent material is lead. As a matter of fact, the latter is known for its ionizing radiation absorbing properties, with a special reference to y rays.

According to another advantageous aspect of the present invention, each arc-shaped body 10, 20 also comprises an inner wall 13, 23 which transversally projects from the inner edge 11B, 21B of its respective flat wall 11, 21. Each inner wall 13, 23 is placed in front of its respective outer wall 12, 22 so as to form a tooth receiving groove.

Obviously, the inner wall 13, 23 is configured for covering the inner surface of the teeth, i.e. that surface of the teeth which faces the inside of mouth. For this purpose, according to a further advantageous aspect of the present invention, it is convenient that the inner wall 13, 23 of a respective arc-shaped body 10, 20 has a length that is equal to, or slightly greater than, the length of the teeth, so as to cover and protect their complete inner surface, as well as part of the gum immediately adjacent to the base of the teeth, to also protect the roots of the teeth.

It is advantageous that the distance of the flat wall 11, 21 between its respective outer edge 11A, 21A and its respective inner edge 11B, 21B be little greater than the thickness of the teeth, for instance 10% or 20% greater. In this way, the device 1 can be held in position thanks to the simple contact of the outer wall 11A, 21A and of the inner wall 11B, 21B with gums.

Preferably, the arc-shaped body 10, 20 is coated with a protection layer 30 (figure 1) configured for protecting the tissues of the oral cavity, in particular from the contact with the ionizing radiation absorbent material. Such coating is preferably arranged on the complete surface of the device 1, both on the outer surfaces and on the inner surfaces of the walls 11, 12, 13 and 21, 22, 23 of each arc-shaped body 10, 20.

Even more preferably the protection layer 30 is made from a thermoplastic material, for instance Ethylene Vinyl Acetate, also known as EVA, and in particular from soft EVA. The protection layer 30 can be colored, for instance by bright and opaque colors, to hide lead, whose color is little pleasant at patients' and in particular children's sight.

In the preferred embodiment of the present invention, which is shown in figure 1, the device 1 comprises two arc-shaped bodies 10, 20, fully identical to each other in their structure. For the sake of simplicity, the elements of the second arc-shaped body 20 that are common to the first arc-shaped body 10 are identified by the same reference numerals, increased by 10.

According to the invention, the arc-shaped bodies 10, 20 are connected to each other at the ends 11C, 21C of their respective flat walls 11, 21. This reciprocal connection makes it possible for the two arc-shaped bodies 10, 20 to move with respect to each other. The two arc-shaped bodies 10, 20 can be manufactured as a single piece (as in the example here shown), or can be removably connected to each other.

The operation of the device 1 according to the present invention is apparent for those skilled in the art on the basis of the previous teachings and is in particular as follows.

The device 1 is produced with dimensions suitable for a patient's mouth.

Before starting an ionizing radiation treatment cycle, the device 1 is placed in the patient's mouth, in particular with the arc-shaped bodies 10, 20 covering the dental arches.

Having installed the device 1, the patient can follow his/her own treatment without any risks of damaging his/her teeth or paradental tissue.

Once the treatment cycle is finished, the device 1 can be easily taken out from the patient's mouth, and subsequently washed out and even sterilized, if necessary.

It is also possible that the device 1 be manufactured as a disposable article, in which case it is simply thrown away after being taken out from a patient's mouth.

## Claims

1. A device (1) for protecting dental arches, comprising two arc-shaped bodies budy (10, 20), each of the two arc-shaped bodies comprising an arc-shaped flat wall (11, 21), configured for covering the ends of the teeth of a respective dental arch, and an outer wall (12, 22) which transversally extends from an outer edge (11A, 21A) of said flat wall (11, 21) and which is configured for covering the outer surface of the teeth of said dental arch, wherein each arc-shaped body (10, 20) is manufactured with a material that comprises an ionizing radiation absorbent material, and wherein each arc-shaped body (10, 20) also comprises an inner wall (13, 23) which transversally projects from an inner edge (11B, 21B) of said flat wall (11, 21), said inner wall (13, 23) being placed in front of said outer wall (12, 22) so as to form a tooth receiving groove, wherein the inner wall (13, 23) of a respective arc-shaped body (10, 20) has a length that is equal to, or slightly greater than, the length of the teeth, so as to cover and protect their complete inner surface, as well as part of the gum immediately adjacent to the base of the teeth, in order to also protect the roots of the teeth, **characterized in that** the two arc-shaped bodies (10, 20) are connected to each other at the ends (11C, 21C) of their respective flat walls (11, 21), said reciprocal connection making it possible for the two arc-shaped bodies (10, 20) to move with respect to each other.

2. The device (1) according to claim 1, wherein each arc-shaped body (10, 20) is manufactured by using a material that is fully made from an ionizing radiation absorbent material.

3. The device (1) according to claim 1 or 2, wherein the ionizing radiation absorbent material is lead.

4. The device (1) according to any claims 1 to 3, wherein each arc-shaped body (10, 20) is coated with a protection layer (30), configured for protecting the tissues of the oral cavity.

5. The device (1) according to claim 4, wherein the protection layer (30) is made from a thermoplastic material.

6. The device (1) according to any claims 1 to 5, wherein the two arc-shaped bodies (10, 20) are manufactured as a single piece.

7. The device (1) according to any claims 1 to 5, wherein the two arc-shaped bodies (10, 20) are removably connected to each other.

## Patentansprüche

1. Vorrichtung (1) zum Schützen von Zahnbögen, zwei bogenförmige Hauptteile (10, 20) umfassend, wobei jeder der zwei bogenförmigen Hauptteile eine bogenförmige ebene Wand (11, 21) umfasst, die dafür gestaltet ist, die Enden der Zähne eines entsprechenden Zahnbogens zu bedecken, und eine Außenwand (12, 22), die sich quer von einem Außenrand (11A, 21A) der ebenen Wand (11, 21) erstreckt und die dafür gestaltet ist, die äußere Oberfläche der Zähne des Zahnbogens zu bedecken, wobei jeder bogenförmige Hauptteil (10, 20) aus einem Material hergestellt ist, das ein ionisierendes, strahlungsabsorbierendes Material umfasst, und wobei jeder bogenförmige Hauptteil (10, 20) außerdem eine Innenwand (13, 23) umfasst, die quer von einem Innenrand (11B, 21B) der ebenen Wand (11, 21) hervorsteht, wobei die Innenwand (13, 23) derart vor der Außenwand (12, 22) platziert ist, dass eine zahnaufnehmende Rille gebildet ist, wobei die Innenwand (13, 23) eines entsprechenden bogenförmigen Hauptteils (10, 20) eine Länge aufweist, die gleich der oder etwas größer als die Länge der Zähne ist, so dass deren komplette Innenfläche bedeckt und geschützt ist, wie auch ein Teil des Zahnfleischs, der unmittelbar an die Basis der Zähne angrenzt, um auch die Wurzeln der Zähne zu schützen,
**dadurch gekennzeichnet, dass** die zwei bogenförmigen Hauptteile (10, 20) an den Enden (11C, 21C) ihrer entsprechenden ebenen Wände (11, 21) miteinander verbunden sind, wobei die gegenseitige Verbindung das Bewegen der zwei bogenförmigen Hauptteile (10, 20) im Verhältnis zueinander ermöglicht.

2. Vorrichtung (1) nach Anspruch 1, wobei jeder bogenförmige Hauptteil (10, 20) unter Verwendung eines Materials hergestellt ist, das vollständig aus einem ionisierenden, strahlungsabsorbierenden Material besteht.

3. Vorrichtung (1) nach Anspruch 1 oder 2, wobei das ionisierende, strahlungsabsorbierende Material Blei ist.

4. Vorrichtung (1) nach einem der Ansprüche 1 bis 3, wobei jeder bogenförmige Hauptteil (10, 20) mit einer Schutzschicht (30) beschichtet ist, die zum Schutz der Gewebe der Mundhöhle gestaltet ist.

5. Vorrichtung (1) nach Anspruch 4, wobei die Schutzschicht (30) aus einem Thermoplastmaterial besteht.

6. Vorrichtung (1) nach einem der Ansprüche 1 bis 5, wobei die zwei bogenförmigen Hauptteile (10, 20) als ein Einzelstück hergestellt sind.

7. Vorrichtung (1) nach einem der Ansprüche 1 bis 5, wobei die zwei bogenförmigen Hauptteile (10, 20) lösbar miteinander verbunden sind.

## Revendications

1. Dispositif (1) pour protéger les arcades dentaires, comprenant deux corps en forme d'arc (10, 20), chacun des deux corps en forme d'arc comprenant une paroi plate en forme d'arc (11, 21), configurée de façon à couvrir les extrémités des dents d'une arcade dentaire respective, et une paroi externe (12, 22) qui fait transversalement saillie d'un bord externe (11A, 21A) de ladite paroi plate (11, 21) et qui est configurée de façon à recouvrir la surface externe des dents de ladite arcade dentaire, dans lequel chaque corps en forme d'arc (10, 20) est fabriqué avec un matériau qui comprend un matériau absorbant les rayonnements ionisants, et dans lequel chaque corps en forme d'arc (10, 20) comprend également une paroi interne (13, 23) qui fait transversalement saillie d'un bord interne (11B, 21B) de ladite paroi plate (11, 21), ladite paroi interne (13, 23) étant placée devant ladite paroi externe (12, 22) de façon à former une rainure réceptrice pour dent, dans lequel la paroi interne (13, 23) d'un corps en forme d'arc (10, 20) respectif possède une longueur qui est égale, ou légèrement supérieure, à la longueur des dents, de façon à recouvrir et protéger la totalité de leur surface interne, ainsi qu'une partie de la gencive immédiatement adjacente à la base des dents, afin d'également protéger les racines des dents, **caractérisé en ce que** les deux corps en forme d'arc (10, 20) sont connectés l'un à l'autre au niveau des extrémités (11C, 21C) de leurs parois plates respectives (11, 21), ladite connexion réciproque permettant aux deux corps en forme d'arc (10, 20) de se déplacer l'un par rapport à l'autre.

2. Dispositif (1) selon la revendication 1, dans lequel chaque corps en forme d'arc (10, 20) est fabriqué à l'aide d'un matériau qui est entièrement constitué d'un matériau absorbant les rayonnements ionisants.

3. Dispositif (1) selon la revendication 1 ou 2, dans lequel le matériau absorbant les rayonnements ionisants est du plomb.

4. Dispositif (1) selon l'une quelconque des revendications 1 à 3, dans lequel chaque corps en forme d'arc (10, 20) est revêtu d'une couche de protection (30), configurée de façon à protéger les tissus de la cavité buccale.

5. Dispositif (1) selon la revendication 4, dans lequel la couche de protection (30) est fabriquée à partir d'un matériau thermoplastique.

6. Dispositif (1) selon l'une quelconque des revendications 1 à 5, dans lequel les deux corps en forme d'arc (10, 20) sont fabriqués en une seule pièce.

7. Dispositif (1) selon l'une quelconque des revendications 1 à 5, dans lequel les deux corps en forme d'arc (10, 20) sont connectés l'un à l'autre de façon amovible.
